# EUROPEAN PATENT SPECIFICATION

(11) **EP 0 686 041 B1**
(45) Date of publication and mention of the grant of the patent: **14.05.1997**
(21) Application number: 94906969.4
(22) Date of filing: 23.02.1994
(51) Int. Cl.: A61K 39/00

(54) **THE USE OF ANTI-HELMINTH VACCINES IN THE CONTROL OF PARASITE DISEASE ASSOCIATED WITH LOSS OF NATURAL IMMUNITY**
DIE VERWENDUNG VON ANTI-HELMINTH IMPFSTOFFEN ZUR KONTROLLE VON PARASITÄREN KRANKHEITEN, DIE MIT EINEM VERLUST DER NATÜRLICHEN IMMUNITÄT ASSOZIIERT SIND
UTILISATION DE VACCINS ANTI-HELMINTHIQUES DANS LA LUTTE CONTRE LES PARASITOSES ASSOCIEES A LA PERTE DE L'IMMUNITE NATURELLE

(30) Priority: 24.02.1993 GB 9303681
(43) Date of publication of application: 13.12.1995
(73) Proprietor: Mallinckrodt Veterinary, Inc., Mundelein Illinois 60060 (US)
(72) Inventor: ROLPH, Timothy Peter Mallinckrodt Veterinary Ltd., Harefield Uxbridge Middlesex UB9 6LS (GB); ANDREWS, Stuart John Mallinckrodt Veterinary Ltd., Harefield Uxbridge Middlesex UB9 6LS (GB)
(74) Representative: Dzieglewska, Hanna Eva
(86) International application number: GB9400354
(87) International publication number: WO9419010

(56) References cited:
- WO-A-90/11086
- WO-A-93/23542
- WO-A-94/02169

## Description

The present invention relates to the use of helminth antigens in preparing vaccines for controlling disease caused by helminth parasites, particularly in domestic animals, and especially to the use of such antigens in controlling parasite disease associated with suppression/alteration of natural immunity e.g. the so-called periparturient rise or type II ostertagiasis.

Helminth parasites are responsible for a wide range of diseases and infestations of domestic and other animals which, leading as they do to loss of production and even animal mortality, are of considerable economic importance. In the case of humans, helminth infections may lead to severe debilitation and death. As regards domestic animals, particular mention may be made of Haemonchus, a blood-feeding nematode which infects the abomasum of ruminants, causing anaemia and weight loss, and which if untreated frequently leads to death, and of the non-blood feeding nematode Ostertagia (Teladorsagia) which causes similar problems in terms of animal mortality and ill-thrift, particularly in cattle and sheep.

Other helminths of economic importance include Trichostrongylus, Cooperia, Chabertia, Oesophagostomum, Nematodirus, Dictyocaulus, and various flukes (eg. Fasciola).

Natural immunity to helminths such as Haemonchus may develop in herds or flocks persistently exposed to helminth-infected pastures, but control of helminth infection has traditionally been accomplished by the use of anthelmintic drugs together with pasture management. An anti-helminth vaccine would have obvious advantages over drugs which require frequent administration and to which resistance may develop, and efforts in recent years have been directed towards immunological means of control.

Most promising results to date have been obtained with proteins extracted from the gut of Haemonchus, which have potential as protective antigens, not only against Haemonchus but also against a range of other helminths. In particular the protein doublet H11OD, found at the luminal surface of the intestine of H. contortus has been shown to confer protective immunity against haemonchosis in sheep (see for example W088/00835).

The so-called periparturient rise, also called the post-parturient rise, presents a particular problem however. This phenomenon refers to the increase in the numbers of helminth (generally nematode) eggs, typically those of a number of different species, including H. contortus, which is observed in the faeces of animals during the latter stages of pregnancy and particularly at around the time of parturition and early stages of lactation and, although not fully understood, is thought to be due to a temporary relaxation in immunity associated with the changes in the circulating levels of the hormone prolactin which occur in the animal during pregnancy or a decrease in immunoglobulin secreted at the gut mucosal surface due to the transfer of immunoglobulin in the plasma to the mammary epithelium followed by its secretion in milk. Reduction in host immunity results in an increased egg output from either the development of recently ingested infective larvae, or from the activation/resumed development of arrested larvae to adults, or both, in turn leading to high levels of eggs (and subsequent generation of infective larvae) on the pasture just at the time when the next generation of animals, and hence new susceptible hosts, is being born thereby ensuring the survival and propagation of the helminth species. The cycle of infection thus continues.

To ensure that susceptible animals are adequately protected and to prevent regular outbreaks of helminth infestations from occurring, it is therefore desirable to break this cycle of infection at the periparturient stage. As mentioned above, natural immunity falls at the critical period and thus far the only means of controlling the periparturient rise has been the administration of anthelmintic drugs, generally prior to tupping. However, during pregnancy, animals usually continue to pick up infective larvae and parasitism may re-establish. A clear need therefore exists for an improved method for controlling the periparturient rise and reducing the subsequent incidence of helminth infection of susceptible young animals in the post-parturient period. Furthermore, loss of natural immunity to gastro-intestinal nematodes in non-pregnant animals arising from seasonal changes in diet, housing and parasite exposure may also result in disease, frequently fatal, for example type II ostertagiasis in cattle.

We have now surprisingly found that a particular regime of vaccination, using hidden antigens obtained from helminths, under the conditions described hereinafter, is successful in reducing helminth egg production in animals suffering from a loss of natural immunity, and in particular in infected pregnant animals and therefore in preventing the periparturient rise and breaking the cycle of infection.

In one aspect, the invention accordingly provides use of a helminth hidden antigen, or antigenic fragments, precursors and functionally-equivalent derivatives or variants thereof having immunogenic activity against one or more helminth parasites, in the manufacture of a vaccine composition for administration to an animal at least once prior to and/or during a period of loss or waning of natural immunity to gastro-intestinal helminth parasites, whereby to stimulate protective immunity against said helminth parasites during the period of compromised natural immunity.

More particularly, the invention provides use of a helminth hidden antigen, or antigenic fragments, precursors and functionally-equivalent derivatives or variants thereof having immunogenic activity against one or more helminth parasites, in the manufacture of a vaccine composition for administration to a female animal at least once during a period prior to tupping and/or up to the beginning of the third trimester of pregnancy, whereby to stimulate protective immunity against said helminth parasites during the periparturient period, and in particular to prevent or reduce the periparturient rise.

As used herein the term "compromised natural immunity" includes loss or waning of natural immunity which leads to ineffective or inadequate protection.

Thus, according to the invention, the vaccine is administered during a period when the immune system of the animal remains competent and serves to establish a memory response to the antigen in question. In the case of periparturient immunisation, preferably, the vaccine may be administered at least once during a period prior to tupping and/or up to the beginning of the second trimester of pregnancy. As will be described in more detail below, it is generally preferable to repeat the immunisation one or more times during pregnancy, consistent with animal husbandry practices.

Waning of natural immunity to gastro-intestinal nematodes can be manifested by:-
1. resumed maturation of arrested larvae;
2. an increase in the rate of establishment of ingested infective larvae;
3. reduced expulsion of existing adult worms;
4. an increased fecundity of established adult populations;

All four of these factors are thought to contribute to the periparturient rise. Insofar as other conditions associated with loss or waning of natural immunity are concerned, for example type II ostertagiasis, the resumed maturation effect is believed to be of primary importance.

Moreover, hypobiotic or immunologically arrested larvae, that is larvae undergoing the seasonal arrestation of development which is typical of certain nematode species, tend to mature in spring around the time of host parturition and this spring rise may also contribute to the periparturient rise. Natural immunity to helminths such as Haemonchus acts mainly on the early L4 larval stage which is the stage at which arrestation occurs in this genus. Once larvae have successfully resumed development they may no longer be controlled by host natural immunity. In contrast, hidden antigens are effective in stimulating a protective immunity against the later larval and established adult stages which have eluded the host's natural immune mechanism.

The effect of the present invention, that is the stimulation of a protective immune response during periods when natural immunity to helminths is compromised, for example, during the usually parasite immune-response-diminished period of host pregnancy which acts to prevent or reduce the periparturient rise, is surprising and unpredictable; earlier studies have shown that vaccination of sheep and cattle using antigens shown by immunoscreening to contribute to the natural immune response, is not effective. In the case of periparturient immunisation, it was not to be expected therefore, that by giving a primary immunisation to the animals before or shortly after tupping optionally followed by a second immunisation during gestation, the immunisation would succeed in eliciting an immune response capable of preventing the periparturient rise. Similarly, it is not to be expected that other conditions associated with loss or waning of natural immunity, such as type II ostertagiasis, could also be controlled by immunisation during the period of compromised natural immunity.

Whilst not wishing to be bound by theory, the effect is believed to be primarily due to the prevention of maturation of hypobiotically or immunologically arrested larvae to adults thereby reducing the level of established infection. A reduction in the numbers of eggs produced by the adult parasites may also play a significant role. This may be of particular importance for helminths such as Ostertagia where maturation of arrested larvae in pregnant animals, or following seasonal climatic changes in both male and female cattle frequently leads to fatal disease, for example type II ostertagiasis.

A further factor of importance in the periparturient effect may be the transfer, via the colostrum to the feeding young, of protective maternal antibodies, induced by immunisation with the hidden antigens, which may also be of significance in reducing the incidence of infection, for example by Haemonchus, in the young animals following ingestion of larvae derived from eggs resulting from the periparturient rise. This is in stark contrast to natural immunity, which is not transferred from mother to offspring.

The term "hidden antigen" as used herein defines those antigens, also known as "cryptic", "covert" or "concealed" antigens, which do not, during the usual course of infection, come into contact with the host's immune system, and which consequently are not recognised by sera from animals which have acquired natural immunity to the parasite in question. Such antigens thus generally derive from those, generally internal, parts of the helminth which are not during infection exposed to the immune system of the host. Thus, for example the helminth gut has been shown to be a particularly rich source of hidden antigens.

A precursor for the antigen in question may be a larger protein which is processed, eg. by proteolysis, to yield the antigen per se. Such precursors may take the form of zymogens ie. inactive precursors of enzymes, activated by proteolytic cleavage, for example analogous to the pepsin/pepsinogen system or the well known zymogens involved in the blood clotting cascade.

Since hidden antigens exert their effects by eliciting the production in the host's blood stream of immune effector molecules, such as antibodies or complement, which are taken up by the parasite when the host's blood is ingested, the hidden antigen concept was initially thought to be limited to blood-feeding parasites. We have shown however that hidden antigens may also be used as the basis of vaccines for non-blood feeding helminth parasites.

The term "immunogenic activity" as used herein defines those antigens and their fragments, precursors, derivatives and variants capable of generating a host-protective immune response ie. a response by the host which leads to generation of immune effector molecules, antibodies or cells which sterilise or reduce the fecundity of, damage, inhibit or kill the parasite and thereby both reduce the parasite egg output and "protect" the host from clinical or sub-clinical disease and loss of productivity.

Such a protective immune response to hidden antigens may most commonly be manifested by the generation of antibodies which are able to inhibit the metabolic function of the parasite, leading to stunting, lack of egg production and/or death. This production of protective, mainly IgG, antibodies which are present in the serum of the host is an important factor distinguishing the type of immune response elicited by hidden antigens from that observed with natural immunity; whereas the hidden antigen based immune effector mechanism is primarily humoral ie. antibody-based, natural immunity to parasites appears to be primarily cell-mediated ie. eosinophils and mast cells which are brought into action to release substances which damage the parasite, the humoral arm of the immune response being less important.

These differences in hidden antigen-induced immunity are thought to be an important contributing factor in the success of hidden antigens in controlling helminth disease during periods of compromised natural immunity e.g. the periparturient rise and type II ostertagiasis in cattle.

As mentioned above, included within the scope of this invention are functionally-equivalent fragments derivatives and variants of helminth hidden antigens. "Functionally equivalent" is used herein to define proteins, including glycoproteins, related to or derived from the native proteins, where the amino acid sequence has been modified by single or multiple amino acid substitution, addition and/or deletion and also sequences where the amino acids have been chemically modified, including by deglycosylation or glycosylation, but which nonetheless retain protective antigenic (immunogenic) activity eg. are capable of raising host protective antibodies and/or functional immunity against helminths. Such functionally-equivalent variants may occur as natural biological variations or may be prepared using known techniques. For example, functionally-equivalent recombinant proteins may be prepared using the known techniques of site-directed mutagenesis, random mutagenesis, or enzymatic cleavage and/or ligation of nucleic acids.

As mentioned above, in periparturient immunisation the hidden antigen-based vaccine composition is administered to the female animals at least once during a period prior to tupping and/or up to the beginning of the third trimester of pregnancy, more preferably up to the beginning of the second trimester. Generally, however the vaccine is administered more than once, for example with a primary injection prior to tupping and one or more boosters during the period up to the beginning of the third, more preferably up to the beginning of the second trimester of pregnancy.

Conveniently, the vaccine may be administered firstly within a period ranging from 60 days before to 20, or more preferably, 10 days after tupping, for example within the 45 days leading to tupping or more preferably within the period of 40 to 5, especially 40 to 10 days before tupping. Whilst administration of the first vaccine injection shortly before tupping is preferred, different husbandry practices on different farms may however dictate that immunisation at different times, for example 6 months before tupping, or even shortly after birth of the young animals, may be more convenient.

The booster administration may be at any time post tupping up to the beginning of the third trimester of pregnancy. For example, a booster may be given one or more times in the period up to the early-second trimester to the early-third trimester of pregnancy.

As discussed above, vaccination with the same immunogen may take place more than once during the relevant period, and different combinations may be appropriate for different animals or vaccines. Alternatively, however the use of a controlled-release antigen delivery system would induce a long-term immune response from a single vaccination (O'Hagan, et al., 1991, Immunology, 73: 239-242). If a primary injection is given more than 6 months before tupping, a further injection within the 6 months leading up to tupping will generally be required. An annual booster injection may suffice in some cases, for example over the winter period, when the risk of infection is low. As an example of a suitable immunisation regime, administration of a H11OD-based vaccine twice at about 40 and 10 days before and about 90 days post tupping has been found to be effective in immunisation of sheep against Haemonchus.

Although immunisation of animals solely during late gestation has been found to be ineffective, where a controlled-release vaccine preparation is not used, one or more booster administrations of the vaccine should preferably be given during gestation up to the beginning of the third trimester, for example for sheep, at around 50-100 days, eg. 45 to 90 days during gestation.

Animals which may benefit from the present invention may be any human or non-human animal, but companion animals, particularly dogs and cats and domestic animals, especially ruminants are preferred. Particular mention may be made of sheep, cattle, deer and goats.

A number of helminth species are associated with the periparturient rise and thus are suitable targets for the vaccine composition. These include most notably species of Haemonchus, Ostertagia, Trichostrongylus, Chabertia, Oesophagostomum, Hyostrongylus, Nematodirus, Toxocara and Cooperia. Hidden antigens may be obtained from a range of such helminths.

Preferred are those antigens, so called "broad spectrum" antigens, which are capable of stimulating host protective immune responses against, in addition to the helminths from which they were isolated, a range of other helminth parasites.

Conveniently, the hidden antigens used according to the invention may be any integral membrane protein isolated from the gut of the helminth, including for example enzymes, structural proteins or any other functional protein required for the maintenance and/or development of the helminth eg. proteins involved in the nutrient-harvesting mechanism of the helminth. Such proteins may include for example enzymes eg. proteases or exo- and endopeptidases involved in degrading ingested protein to free amino acid.

Suitable hidden antigens include H11OD, mentioned above, the preparation of recombinant forms of which is described in WO93/23542. Mention may also be made of the protein complex H45 described in W090/11086 and of the antigens described in WO94/02169. In the latter case, such antigens include integral membrane proteins, having a native localisation in the parasite gut and characterised by being capable of binding to pepstatin, and to wheatgerm lectin and peanut lectin and other lectins having specificity for β-linked N-acetylgalactosamine. Such antigens may be proteolytic enzymes, particularly enzymes having aspartyl protease and/or neutral endopeptidase activity. Particular mention may be made of the antigens H-gal-GP and O-gal-GP, which are described in detail in WO94/02169.

The antigens and their fragments, precursors and functionally-equivalent variants and derivatives may be native antigens isolated directly from helminths, or may be prepared by recombinant DNA technology using standard techniques such as those described for example by Sambrook et al., 1989 (Molecular Cloning, a laboratory manual 2nd Edition, Cold Spring Harbour Press), or by chemical synthesis such as by the well-known Merrifield solid phase synthesis procedure.

The vaccine composition administered to the animal may be polyvalent, containing a range of antigenic components, for example which are active against a range of helminth species.

A vaccine composition may be prepared according to the invention by methods well known in the art of vaccine manufacture. Traditional vaccine formulations may comprise one or more antigens or antibodies according to the invention together, where appropriate, with one or more suitable adjuvants eg. aluminium hydroxide, potash alum, saponin or derivatives thereof, muramyl dipeptide, mineral or vegetable oils, NAGO, Novasomes or non-ionic block co-polymers, DEAE dextran or controlled release antigen delivery systems such as biodegradable microparticles, in the presence of one or more pharmaceutically acceptable carriers or diluents. Suitable carriers include liquid media such as saline solution appropriate for use as vehicles to introduce the peptides or polypeptides into an animal or patient. Additional components such as preservatives may be included.

An alternative vaccine formulation may comprise a virus or host cell eg. a microorganism which may be live, killed or attenuated, having inserted therein a nucleic acid molecule (eg. a DNA molecule) for stimulation of an immune response directed against polypeptides encoded by the inserted nucleic acid molecule.

Administration of the vaccine composition may take place by any of the conventional routes, eg. orally or parenterally such as by intramuscular, sub-cutaneous, or intradermal injection. Injection is generally preferred.

The invention will now be discussed in more detail with particular reference to the prevention of the periparturient rise of Haemonchus in sheep using the hidden antigen H110D. While not limiting generality of the invention defined above it will be understood that the use of Haemonchus antigens to prevent the periparturient rise in Haemonchus egg output in sheep represents a preferred aspect of the invention. In the following Examples, the Figures represent:

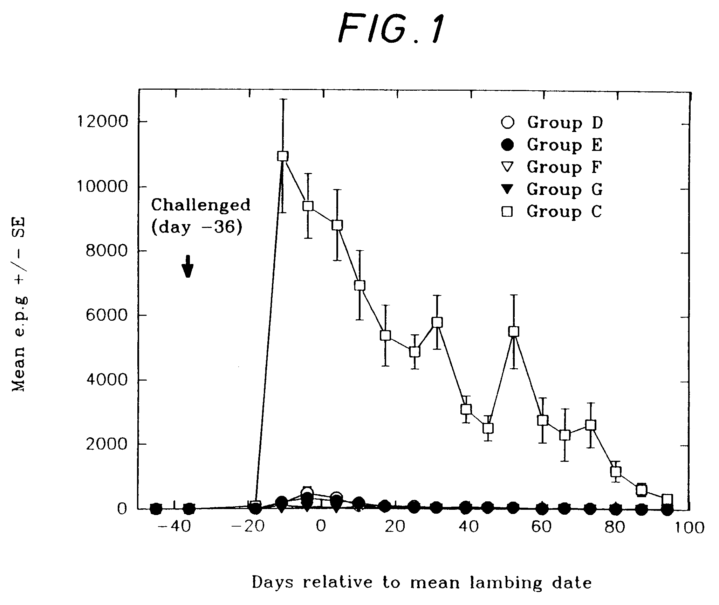

Figure 1 shows the mean faecal egg counts (Thousand eggs per gram) over time in vaccinated and non-vaccinated pregnant and non-pregnant ewes challenged with infective Haemonchus larvae at 110 days gestation.
   - □ Group D: (17 pregnant ewes vaccinated with H110D and the clostridial vaccine COVEXIN 8)
   - + Group E: (18 pregnant ewes vaccinated with H110D)
   - ◇ Group F: (5 non-pregnant ewes vaccinated with H110D and COVEXIN 8)
   - △ Group G: (3 non-pregnant ewes vaccinated with H110D)
   - x Group C: (17 pregnant ewes vaccinated with ferritin as control)
   The arrow indicates the time of challenge; and

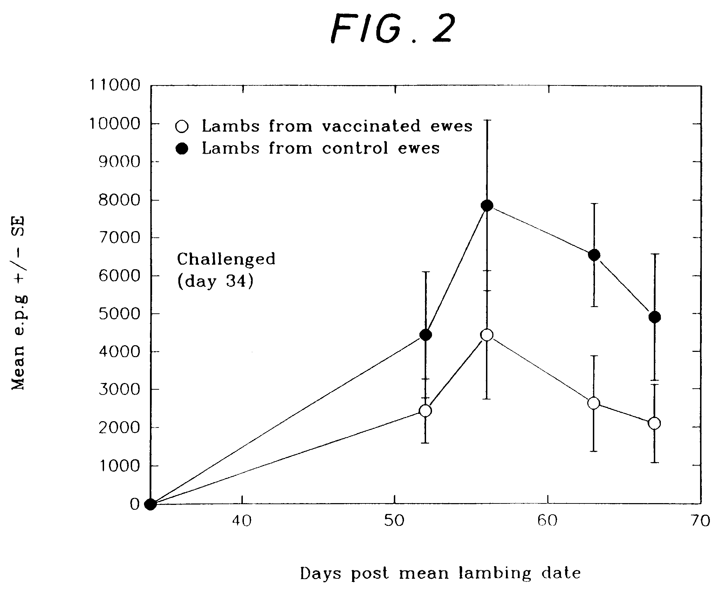

   Figure 2 shows the protection of lambs against H. contortus challenge at 5 weeks of age by colostral transfer of antibodies to H110D (faecal egg counts (thousand eggs per gram) over time (days post mean lambing date)). ∘lambs from vaccinated ewes; •lambs from control ewes.

### EXAMPLE 1

This Example aimed
(i) To assess the efficacy of an H110D vaccine in controlling the worm egg output associated with H. contortus infection in ewes during the periparturient period.
(ii) To monitor antibody response in both pregnant and non-pregnant ewes to assess the impact of pregnancy on the immunogenicity of the antigen H110D.
(iii) To assess the effect of co-administering the clostridial vaccine COVEXIN 8 on the efficacy of the H11 vaccine. The compatibility of existing bacterial vaccines with novel parasite vaccines is a prerequisite to their adoption in practice.

### Introduction

This study assessed the efficacy of the H11OD vaccine in controlling the worm egg output from gimmers (second year ewe lambs) vaccinated twice preconception and boosted during pregnancy, and receiving an artificial challenge of H. contortus infective larvae during the last trimester of gestation. These gimmers have been compared with age matched vaccinated non-pregnant controls.

| TIMETABLE (Main Dates) | | |
|---|---|---|
| Date | Day Number | Action |
| Early September | -50 | Gimmers dosed with levamisole (NILVERM GOLD) out to pasture. |
| | -40 | Group randomisation and primary vaccinations. |
| | -10 | Secondary Vaccinations. |
| | - 8 | Rams introduced for approximately three weeks. |
| | 0 | (Group mean date of conception) |
| | 80 | All gimmers dosed with ivermectin (ORAMEC DRENCH) and housed. |
| | 90 | Tertiary Vaccinations. |
| | 110 | All gimmers received ca.10,000 H. contortus infective larvae. |
| | | Fortnightly faecal sampling started. |
| March/April | 147 | Lambing started. |
| July/August | 267 | Lambs weaned. |

### Methods and Experimental Design

Sixty gimmers, dosed with levamisole (NILVERM GOLD) to remove any established worm burden, were allowed access to paddocks from early September 1991. Following a minimum period of seven days acclimatisation the gimmers were allocated randomly into three groups A, B and C, comprising 35, 8 and 17 gimmers respectively. The gimmers in groups A and B were vaccinated with H110D (day -40), whilst those in group C were vaccinated with ferritin to act as controls. Thirty days later (day - 10) the gimmers received their second vaccinations and then immediately were divided into further groups:-Those gimmers in group A were divided to form two new groups, D and E, whilst those in group B likewise formed groups F and G. Rams were introduced during the last week in October with those animals in groups C, D and E to allow for a group mean date of conception as close as possible to day 0 and a planned lambing date of the last week of March, first week of April. The gimmers in groups F and G were not put to the ram so as to act as vaccinated non-pregnant controls. After approximately six weeks the rams were removed from the paddocks, and all gimmers allowed to graze together as one flock. On day 80 all the gimmers were dosed with ivermectin (ORAMEC DRENCH) in order to remove all adult worms and arrested larvae, and housed. A tertiary vaccination was with H11OD administered to all gimmers in groups D, F, E and G on day 90 (that is 90 days gestation ± 10 days). The gimmers in groups D and F also received injection of COVEXIN 8 at this time. At 110 days gestation ± 10 days, all animals received a challenge of ca. 10,000 H. contortus infective larvae. Nematode numbers were monitored by faecal sampling. The absence of possible suppression of the immune response to the hidden antigen H110D during pregnancy was confirmed by monitoring antibody levels.

| Summary of Animal Grouping: | | |
|---|---|---|
| Group Identification: | Vaccination Status: | Pregnant/non-pregnant |
| Group A | Vaccinated twice preconception (H110D) | Pregnant |
| Group D | H110D + COVEXIN 8 at 90 days gestation | " |
| Group E | H110D at 90 days gestation | Pregnant |
| Group B | Vaccinated (H110D) } | Non-Pregnant |
| Group F | H110D + COVEXIN 8 } | " |
| Group G | H110D } | " |
| Group C | Vaccinated (ferritin)} | Pregnant |

| | | |
|---|---|---|
| } = Time matched vaccination to groups D and E | | |

### TEST MATERIALS

### Description

Haemonchus contortus (H110D) vaccine was supplied and prepared by the Institute of Animal Physiology and Genetics Research, Babraham, Cambridge. The adjuvant system for the primary vaccination was Freund's complete adjuvant/aluminium hydroxide (FCA/A1(OH)₃) and Freund's incomplete adjuvant/aluminium hydroxide (FIA/Al(OH)₃) for both the secondary and tertiary vaccinations. Horse ferritin control vaccine was supplied and prepared by the Institute of Animal Physiology and Genetics Research, Babraham, Cambridge. The adjuvant system used was the same as above.

### PARASITE CHALLENGE

### Species/Strain

A benzimidazole-resistant strain of H.contortus (strain H/CR) was used in the study. The strain was obtained as third stage larvae from the Central Veterinary Laboratory, Weybridge. Benzimidazole-resistance had been confirmed on egg hatch assay and controlled test (Cawthorne RJG and Cheong FH 1984, Veterinary Record 114: 562).

### Larval Culture

Sufficient numbers of third stage larvae were produced by passage through a worm free lamb. Faeces containing eggs were collected and cultured to the infective stage. Infective larvae were recovered and cleaned using a Baermann apparatus.

### Infective Doses

Inocula of ca.10,000 infective larvae were prepared by a standard dilution technique. Larvae were administered by mouth using clean plastic tubes.

### Challenge Procedure

To allow a controlled challenge in the last trimester of gestation all gimmers were treated with ivermectin (ORAMEC DRENCH) to remove any accumulated worm burden, and housed immediately on day 80. All gimmers received a challenge on day 110 of gestation of ca. 10,000 H. contortus infective larvae, which was used to mimic, in a controlled manner the development of a periparturient rise.

### EXPERIMENTAL PROCEDURES

### Control of Bias

All gimmers were randomised into groups on the basis of bodyweight at the designated time.

### Vaccination Procedure

The gimmers in group A and B were vaccinated by intramuscular injection into the hind legs (1ml per leg, total of 2mls) with 150ug of H. contortus antigen (H110D) on day -40 and again into the front legs (1ml per leg, total of 2mls) on day -10. On day 90, following the formation of groups D, E, F and G all the gimmers in these four groups received a third shot of the vaccine by intramuscular injection into the hind legs (procedure as for primary). Coincidentally, those gimmers in groups D and F were injected subcutaneously in the neck (as per manufacturer's data sheet) with the clostridial vaccine COVEXIN™ 8. The gimmers in group C remained as controls and received injections of horse ferritin (same route and volume as H110D vaccine) at days -40, -10 and 90 and COVEXIN™ 8 (subcutaneously) at day 90.

### ASSESSMENT OF PROTECTION

### Faecal Egg Counts

Faecal samples were taken from all gimmers via the rectum at weekly intervals commencing approximately six weeks prior to lambing, until the conclusion of the study. Faecal egg counts were estimated by a modified McMaster method accurate to 10 eggs per gram (epg) of faeces. Faecal samples were cultured fortnightly for 7 days at 27°C for larval identification, and confirmation of purity of challenge.

### Antibody Response following Vaccination with ConAH11

Blood samples were taken from a jugular vein of all gimmers into plain blood containers at fortnightly intervals post primary vaccination. Pre-bleeds were collected on the day of primary immunisation for reference sera. Antibody levels were estimated by an ELISA method.

### RESULTS

The results of the study are shown in Figure 1. This clearly shows that non-vaccinated pregnant ewes (Group C) produce high levels of nematode eggs around the time of lambing following artificial challenge. All vaccinated ewes however, produce substantially reduced numbers of eggs in their faeces. Protection was not reduced in ewes which received co-administered clostridial vaccine.

Table 1 shows the results of ELISA tests to determine antibody levels of the animals in the study. It can be seen that all vaccinated ewes exhibited higher antibody levels than the non-vaccinated controls. Antibody levels in vaccinated pregnant ewes were similar to those in the non-pregnant vaccinates, confirming the lack of suppression of antibody response to the hidden antigens. Co-administration of the clostridial vaccine COVEXIN™ 8 did not affect the antibody levels.

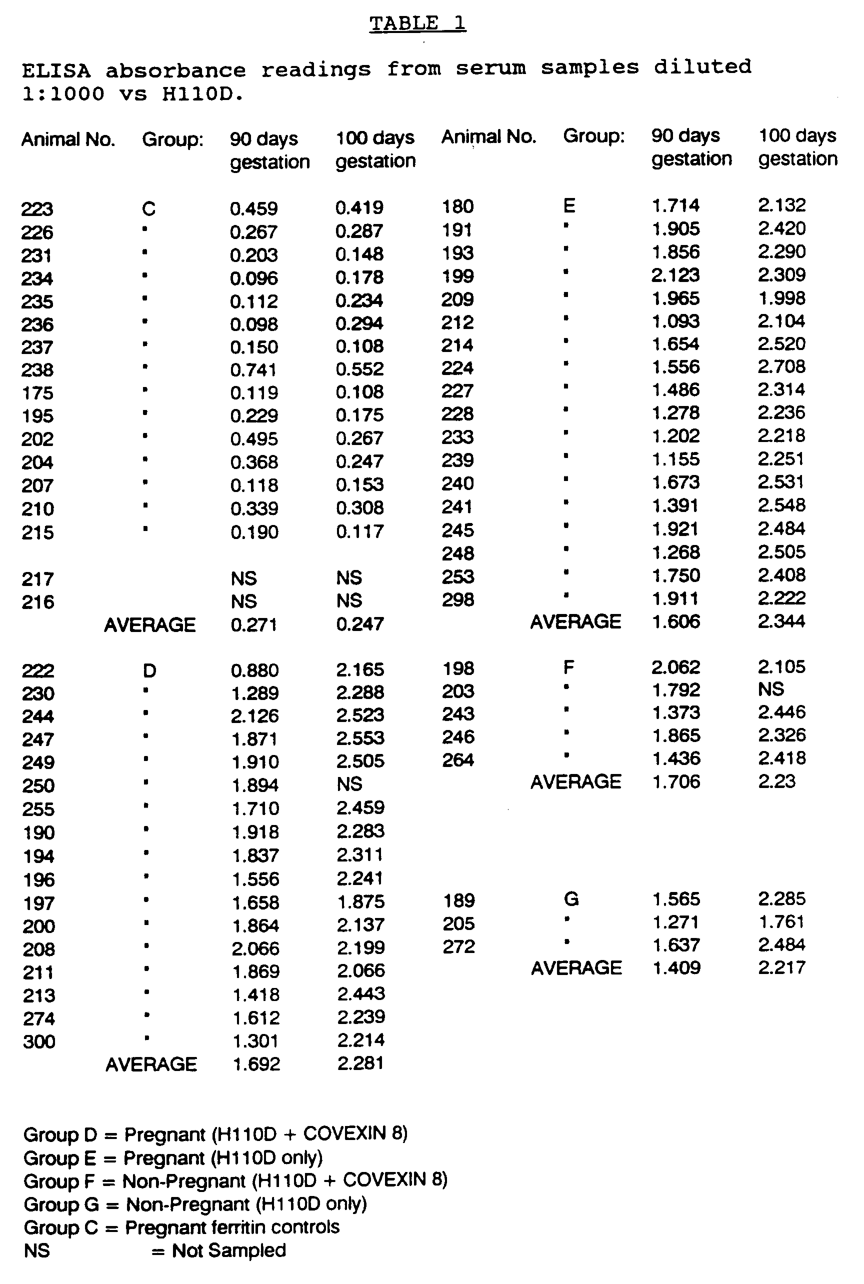

### EXAMPLE 2

### Colostral Transfer of H11OD Mediated Immunity

### Trial Rationale

To estabish that protection could be conferred on young lambs by colostral transfer. Accordingly, protection was determined in lambs, ca. 5 weeks old, suckling on ewes which had been immunised with H110D, by reference to lambs from non-immunised ewes. Protection was determined as reduction in faecal egg output and worm burden following bolus challenge.

### Trial Design

The trial design is summarised:

**Table 2**

| Group | Number of Lambs Challenged | Challenge at 5 Weeks of Age (L₃ Larvae) |
|---|---|---|
| Lambs from Ewes Vaccinated with H11OD during pregnancy | 6 | 3,000 |
| Lambs from Ewes not Vaccinated with H11OD during pregnancy | 6 | 3,000 |

Full details of immunisation of pregnant ewes with H110D are described in Example 1. Ten lambs born from the H110D immunised ewes, together with ten from the control ewes, were selected on a first caught basis at 2-3 weeks old. Antisera from these was screened by ELISA for anti-H110D antibody level. Six lambs with the highest antibody levels were selected from the H110D immunised ewe group. Six control lambs were selected to give a matching age. These two groups of lambs were challenged with 3,000 L₃H. contortus larvae (H/CR strain) at 4-5 weeks. Faecal egg counts were monitored from 8-11 weeks, and worm burden enumerated following necropsy at 11 weeks.

### Results

Colostral transfer of anti-H110D antibodies was confirmed by their high levels in the six lambs from ewes immunised with H110D, see Table 3. In contrast there were no anti-H110D antibodies detected in lambs from control ewes.

**Table 3**

| Age of lambs (weeks) | 2/3 | 3/4 | 7/8 | 8/9 | 10/11 |
|---|---|---|---|---|---|
| | Mean optical density at 405 mm | | | | |
| Lambs from Ewes Immunised with H110D | 1.5 | 1.6 | 0.9 | 0.9 | 0.6 |
| Lambs from Control Ewes | n.d | n.d | n.d | n.d | n.d |
| n.d. = Not detected The dilution of Sera was 1/1,000 | | | | | |

Total faecal egg count from 8 to 11 weeks of age was ca. 50% lower (P<0.05) in lambs from immunised versus control ewes (see Fig. 2.), consistent with fewer adult female worms (see below). Mean worm burdens, 5 weeks after challenge, were:

**Table 4**

| Group | Mean Worm Burden | % Protection | Mean Female/Male Ratio |
|---|---|---|---|
| Lambs from Ewes Immunised with H110D | 625 | 30.0 | 0.35 |
| Lambs from Control ewes | 892 | | 1.15 |

The reduction in worm burden was not significant (P>0.05). However there was a marked reduction in female/male ratio (P<0.05), a characteristic effect of H110D immunisation in more mature lambs.

### Summary

Lambs born and reared on ewes which had been immunised with H110D have substantial antibody levels to H110D derived from colostral transfer.
This antibody confers protection against faecal egg output and worm burden arising from a bolus challenge at 5 weeks of age.

## Claims

1. Use of a helminth hidden antigen, or antigenic fragments, precursors and functionally-equivalent derivatives or variants thereof having immunogenic activity against one or more helminth parasites, in the manufacture of a vaccine composition for administration to an animal at least once prior to and/or during the periparturient period, whereby to stimulate protective immunity against said helminth parasites during the periparturient period.

2. Use as claimed in claim 1, in the manufacture of a vaccine composition for administration to a female animal at least once during a period prior to tupping and/or up to the beginning of the third trimester of pregnancy, whereby to stimulate protective immunity against said helminth parasites during the periparturient period.

3. Use as claimed in claim 2, to prevent or reduce the periparturient rise.

4. Use as claimed in claim 2 or claim 3, wherein said vaccine composition is administered to the female animal at least once prior to tupping and at least once during gestation up to the beginning of the third trimester of pregnancy.

5. Use as claimed in claim 4, wherein said vaccine composition is administered to the female animal at least once prior to tupping and at least once during gestation up to the beginning of the second trimester of pregnancy.

6. Use as claimed in claim 1, to combat type II ostertagiasis.

7. Use as claimed in any of claims 1 to 6 wherein said helminth hidden antigen is an integral gut membrane protein.

8. Use as claimed in claim 7, wherein said helminth antigen is an enzyme or transport protein.

9. Use as claimed in claim 8, wherein said helminth antigen is a protease or exo- or endopeptidase.

10. Use as claimed in claim 9, wherein said helminth antigen is selected from antigens H110D, H45, H-gal-GP and O-gal-GP, or an antigenic fragment thereof or precursor therefor.

11. Use as claimed in any one of claims 1 to 10, wherein said animal is a ruminant.

12. Use as claimed in any one of claims 1 to 10, wherein said animal is selected from sheep, cattle, deer, goats and dogs.

13. Use as claimed in any one of claims 1 to 12, whereby to stimulate protective immunity against helminths selected from Haemonchus, Ostertagia, Trichostrongylus, Chabertia, Oesophagostomum, Hyostrongylus, Nematodirus, Toxocara and Cooperia.

14. Use as claimed in any one of claims 1 to 13, wherein said vaccine composition is in the form of a controlled-release preparation.

15. Use as claimed in any one of claims 1 to 5 and 7 to 14, of the antigen H110D or an antigenic fragment thereof for the manufacture of a vaccine composition for control of the periparturient rise of Haemonchus faecal egg output in sheep.

16. Use as claimed in claim 15, wherein said vaccine composition is administered to female sheep at about 40 to 10 days before tupping and about 90 days post tupping.

## Patentansprüche

1. Verwendung eines verborgenen Helminthen-Antigens oder antiger Fragmente, Vorstufen- oder funktionaläquivalenter Derivate oder Varianten davon mit immunogener Aktivität gegen einen oder mehrere Helminthen-Parasiten bei der Herstellung einer Impfstoffzusammensetzung zur Verabreichung an ein Tier mindestens einmal vor und/oder während der periparturienten Periode, wodurch eine protektive Immunität gegen die Helminthen-Parasiten während der periparturienten Periode stimuliert wird.

2. Verwendung nach Anspruch 1 bei der Herststellung einer Impfstoffzusammensetzung zur Verabreichung an ein weibliches Tier mindestens einmal während eines Zeitraums vor dem Begatten und/oder bis zum Beginnen des dritten Trimesters der Schwangerschaft, wodurch eine protektive Immunität gegen die Helminthen-Parasiten während der pariparturienten Periode stimuliert wird.

3. Verwendung nach Anspruch 2 zur Verhinderung oder Reduzieren des periparturienten Anstiegs.

4. Verwendung nach Anspruch 2 oder 3, worin die Impfstoffzusammensetzung an weibliche Tiere mindestens einmal vor dem Begatten und mindestens einmal während der Gestation bis zum Beginn des dritten Trimesters der Schwangerschaft verabreicht wird.

5. Verwendung nach Anspruch 4, worin die Impfstoffzusammensetzung an das weibliche Tier mindestens einmal vor dem Begatten und mindestens einmal während der Gestation bis zum Beginn des zweiten Trimesters der Schwangerschaft verabreicht wird.

6. Verwendung nach Anspruch 1 zur Bekämpfung von Ostertagiasis vom Typ II.

7. Verwendung nach einem der Ansprüche 1 bis 6, worin das verborgene Helminthen-Antigen ein integrales Darmmembran-Protein ist.

8. Verwendung nach Anspruch 7, worin das Helminthen-Antigen ein Enzym oder ein Transportprotein ist.

9. Verwendung nach Anspruch 8, worin das Helminthen-Antigen eine Protease oder Exo- oder Endopeptidase ist.

10. Verwendung nach Anspruch 9, worin das Helminthen-Antigen ausgewählt ist aus den Antigenen H110D, H45, H-gal-GP und O-gal-GP oder einem antigenen Fragment davon oder einer Vorstufe davon.

11. Verwendung nach einem der Ansprüche 1 bis 10, worin das Tier ein Wiederkäuer ist.

12. Verwendung nach einem der Ansprüche 1 bis 10, worin das Tier ausgewählt ist aus Schafen, Rindern, Wild, Ziegen oder Hunden.

13. Verwendung nach einem der Ansprüche 1 bis 12, zur Stimulierung einer protektiven Immunität gegen Helminthen, ausgewählt aus **Haemonchus, Ostertagia,** **Trichostrongylus, Chabertia, Oesophagostomum, Hyostrongylus, Nematodirus, Toxocara und Cooperia.**

14. Verwendung nach einem der Ansprüche 1 bis 13, worin die Impfstoffzusammensetzung in Form einer Präparation mit kontrollierter Freisetzung vorliegt.

15. Verwendung nach einem der Ansprüche 1 bis 5 und 7 bis 14 des Antigens H110D oder eines antigenen Fragments davon zur Herstellung einer Impfstoffzusammensetzung zur Kontrolle des periparturienten Anstiegs des Ausstoßes von **Haemonchus-**Eiern im Kot von Schafen.

16. Verwendung nach Anspruch 15, worin die Impfstoffzusammensetzung an weibliche Schafe ca. 40 bis 10 Tage vor dem Begatten und ca. 90 Tage nach dem Begatten verabreicht wird.

## Revendications

1. Utilisation d'un antigène caché d'helminthe, ou des fragments, des précurseurs antigéniques et des dérivés ou variants de celui-ci fonctionnellement équivalents ayant une activité immunogénique contre un ou plusieurs parasites helminthes, dans la fabrication d'une composition de vaccin pour l'administration à un animal au moins une fois avant et/ou pendant la période périparturiente, d'où il résulte la stimulation de l'immunité protectrice contre lesdits parasites helminthes pendant la période périparturiente.

2. Utilisation selon la revendication 1, dans la fabrication d'une composition de vaccin pour l'administration à un animal femelle au moins une fois pendant une période précédant l'accouplement et/ou une période allant jusqu'au début du troisième trimestre de la grossesse, d'où il résulte la stimulation de l'immunité protectrice contre lesdits parasites helminthes pendant la période périparturiente.

3. Utilisation selon la revendication 2, pour empêcher ou réduire l'accroissement en période périparturiente.

4. Utilisation selon la revendication 2 ou la revendication 3, dans laquelle ladite composition de vaccin est administrée à un animal femelle au moins une fois avant l'accouplement et au moins une fois pendant la gestation jusqu'au début du troisième trimestre de la grossesse.

5. Utilisation selon la revendication 4, dans laquelle ladite composition de vaccin est administrée à l'animal femelle au moins une fois avant l'accouplement et au moins une fois pendant la gestation jusqu'au début du deuxième trimestre de la grossesse.

6. Utilisation selon la revendication 1, pour combattre l'ostertagiasis de type II.

7. Utilisation selon l'une quelconque des revendications 1 à 6 dans laquelle ledit antigène caché d'helminthe est une protéine membranaire intégrale d'intestin.

8. Utilisation selon la revendication 7, dans laquelle ledit antigène d'helminthe est une protéine enzymatique ou de transport.

9. Utilisation selon la revendication 8, dans laquelle ledit antigène d'helminthe est une protéase, une exoprotéase ou une endoprotéase.

10. Utilisation selon la revendication 9, dans laquelle ledit antigène d'helminthe est choisi dans le groupe des antigènes H110D, H45, H-gal-GP et O-gal-GP, ou un fragment antigénique de ceux-ci ou un précurseur de ceux-ci.

11. Utilisation selon l'une quelconque des revendications 1 à 10, dans laquelle ledit animal est un ruminant.

12. Utilisation selon l'une quelconque des revendications 1 à 10, dans laquelle ledit animal est choisi dans le groupe constitué par les espèces ovine, bovine, caprine et canine et par celle des cervidés.

13. Utilisation selon l'une quelconque des revendications 1 à 12, d'où il résulte la stimulation de l'immunité protectrice contre les helminthes choisis dans le groupe constitué par Haemonchus, Ostertagia, Trichostrongylus, Chabertia, Oesophagostomum, Hyostrongylus, Nematodirus, Toxocara et Cooperia.

14. Utilisation selon l'une quelconque des revendications 1 à 13, dans laquelle ladite composition de vaccin est sous la forme d'une préparation à libération commandée.

15. Utilisation selon l'une quelconque des revendications 1 à 5 et 7 à 14, de l'antigène H110D ou un fragment antigénique de celui-ci dans la fabrication d'une composition de vaccin pour commander l'accroissement en période périparturiente de la production d'oeufs fécaux d'Haemonchus chez les ovins.

16. Utilisation selon la revendication 15, dans laquelle ladite composition de vaccin est administrée à la brebis entre 10 et 40 jours environ avant l'accouplement et 90 jours environ après l'accouplement.
